# EUROPÄISCHE PATENTSCHRIFT

(11) **EP 1 889 585 B1**
(45) Veröffentlichungstag und Bekanntmachung des Hinweises auf die Patenterteilung: **25.03.2009**
(21) Anmeldenummer: 06017265.7
(22) Anmeldetag: 18.08.2006
(51) Int. Cl.: A61B 19/00

(54) **Adapter zur Anbringung einer Referenzanordnung an ein medizinisches Instrument, das eine funktionale Richtung bzw. Ebene aufweist**
Adapter for mounting a reference array to a medical tool, which has respectively a functional direction or plane
Adaptateur pour appliquer une installation de référence au instrument médical, qui a respectivement une direction ou un plan fonctionnel

(43) Veröffentlichungstag der Anmeldung: 20.02.2008
(73) Patentinhaber: BrainLAB AG, 85622 Feldkirchen (DE)
(72) Erfinder: Wagner, Benjamin, 81669 München (DE); Neubauer, Timo, 85586 Poing (DE); Schubert, Mario, 85586 Poing (DE)
(74) Vertreter: Rögner, Jürgen

(56) Entgegenhaltungen:
- DE-A1- 19 813 383
- US-A1- 2003 225 329

## Beschreibung

Die Erfindung betrifft einen Adapter zur Anbringung einer Referenzanordnung an ein medizinisches Instrument, das eine funktionale Ebene aufweist. Solche Referenzanordnungen dienen dazu, ein Instrument für ein medizintechnisches Tracking- bzw. Navigationssystem sichtbar und verfolgbar zu machen. Auch kann anhand einer speziellen Anordnung von Markern innerhalb der Referenzanordnung eine Identifizierung jeder einzelnen Referenzanordnung und damit auch jedes einzelnen Instruments vorgenommen werden. Es ist einerseits bekannt, Instrumente mit an ihnen fest angeordneten Referenzanordnungen auszustatten, andererseits werden in einigen Fällen besonders ausgestaltete Schnittstellen an Instrumenten vorgesehen, an denen dann Referenzanordnungen mit einem Adapter befestigt werden. Diese Adapter weisen eine Halterung für die Referenzanordnung und einen Instrumentenangriff auf, wobei der Instrumentenangriff speziell so ausgestaltet ist, dass er mit der Schnittstelle am Instrument zusammenwirken kann. Das Dokument US 2003/0225329 stellt den nächsten Stand der Technik dar.

Um die Navigation durchführen zu können, müssen die Instrumente den verwendeten Referenzanordnungen zugeordnet und auch bezüglich ihrer äußeren Gestalt in einem Speicher des Navigationssystems hinterlegt werden. Wenn dies der Fall ist, kann das Navigationssystem das Instrument anhand der Referenzanordnung identifizieren und weiß auch, wo sich bestimmte funktionelle Bereiche des Instruments, beispielsweise die Instrumentenspitze, zu jedem Zeitpunkt befinden. Nachteilig wirkt sich hierbei aus, dass man entweder eine große Datenbank mit vorkalibrierten Instrumenten und Referenzanordnungen pflegen und bereitstellen muss oder aber jedes Instrument, das an einer Schnittstelle mit einem Referenzadapter versehen wird, einerseits dem Referenzadapter zugeordnet und andererseits der Form nach kalibriert werden muss, d.h. es muss vor der Benutzung festgestellt werden, wo die Spitze des Instruments liegt. Außerdem ist der Anbau von Referenzanordnungen auf solche Instrumente beschränkt, die mit einer entsprechenden Adapter-Schnittstelle versehen sind.

Es ist die Aufgabe der vorliegenden Erfindung, einen Adapter zur Anbringung einer Referenzanordnung an einem medizinischen Instrument bereitzustellen, der die oben beschriebenen Nachteile überwindet. Insbesondere soll es für eine bestimmte Art von Instrumenten ermöglicht werden, jedwede Referenzanordnung anzubringen, wobei das Instrument im Tracking- bzw. Navigationsumfeld zur Verfügung stehen kann, ohne notwendigerweise vorher in einer Datenbank registriert oder kalibriert zu werden. Diese Aufgabe wird erfindungsgemäß durch einen Adapter gemäß dem Patentanspruch 1 gelöst. Die Unteransprüche definieren bevorzugte Ausführungsformen der Erfindung.

Bei dem erfindungsgemäßen Adapter ist der Instrumentenangriff ein größen-und/oder bedingt form-anpassbarer Klemmangriff und die Referenzanordnungs-Halterung und der Instrumentenangriff sind durch eine selbstausrichtende Kopplungsmechanik miteinander verbunden. Diese Kopplungsmechanik bringt die Referenzanordnung beim Anbringen des Adapters an dem Instrument in eine vorbestimmte Lage relativ zu einer funktionalen Instrumentenrichtung bzw. -ebene.

Mit anderen Worten macht die vorliegende Erfindung sich die Erkenntnis zu Nutze, dass es für manche Instrumente nicht unbedingt notwendig ist, die Lage und Position der funktionellen Instrumententeile vollständig zu kennen. Es ist vielmehr in manchen Fällen durchaus ausreichend, wenn die Referenzanordnung lediglich die Ausrichtung einer bestimmten Richtung bzw. Ebene als Information für das Navigationssystem liefert, und eine solche funktionelle Instrumentenrichtung bzw. -ebene kann beispielsweise eine für das Instrument definierbare Richtung bzw. Ebene sein, welche die Arbeitsrichtung des Instruments bestimmt. Wenn also ein Instrument während der Anwendung in einer bestimmten Richtung z.B. vor- und zurückgeschoben werden soll, genügt es oft, wenn man diese Richtung einhält. Wenn erfindungsgemäß der Adapter die Referenzanordnung in eine vorbestimmte Lage zu einer solchen funktionellen Instrumentenrichtung bzw. -ebene bringt, kann durch die Navigation festgestellt werden, ob die Ebene bzw. die Richtung bei der Anwendung eingehalten wird. Der große Vorteil liegt dabei darin, dass es nicht mehr notwendig ist, die Abmessungen des Instruments in einer Datenbank zu hinterlegen, die Navigation aber trotzdem durchführbar ist. Dies macht es in der Praxis denkbar, Instrumente zu navigieren, ohne deren technische Daten mit dem Navigationssystem auszutauschen, und damit bildet der erfindungsgemäße Adapter eine Art Universaladapter für eine große Anzahl von Instrumenten, die auch ohne weiteres von verschiedenen Herstellern stammen können.

Bei einer bevorzugten Ausführungsform der Erfindung ist das Instrument eine Knochen-Reibahle, und die funktionale Ebene ist die Längsmittelebene der Reibahle, insbesondere des Reibahlengriffes. Die Erfindung stellt über diese Ausführungsform einen Universaladapter für Referenzanordnungen bereit, die an Reibahlen zu befestigen sind. Damit kann der Anteversionswinkel während der Verwendung der Reibahle beim Ausräumen eines Oberschenkelknochens navigiert und verifiziert werden, beispielsweise bei der Durchführung einer Hüftersatz-Operation. Ein solcher Universal-Reibahlenadapter ermöglicht die Navigation und Verifikation des Anteversionswinkels mit Reibahlen, deren Abmessungen weder als Datensatz bekannt sein müssen, noch durch Kalibrierungsvorgänge ermittelt werden müssen. Weil der Adapter es gestattet, die Referenzanordnung in vorbestimmter Lage an dem Griff der Reibahle anzuordnen, ist nach der Anbringung der Referenzanordnung die Längsmittelebene der Reibahle bekannt, und die navigationsunterstützte Anwendung kann beginnen. Es wirkt sich im Rahmen der vorliegenden Erfindung vorteilhaft aus, wenn das Instrument, an dem der Adapter befestigt werden soll, Angriffsflächen hat, die symmetrisch zur funktionalen Instrumentenrichtung bzw. zur funktionalen Instrumentenebene liegen.

Bei einer bevorzugten Ausführungsform der vorliegenden Erfindung weist der Instrumentenangriff zwei gegeneinander wirkende Angriffselemente auf, und die Wirkrichtung des Instrumentenangriffs steht in jeder Stellung der Angriffselemente in einem gleichen Positionsverhältnis zur Ausrichtung der Referenzanordnung. Mit anderen Worten wird die Kraftrichtung für das Halten des Adapters am Instrument immer im selben Verhältnis zur Referenzanordnungs-Ausrichtung gehalten, egal ob die Klemme wenig oder stärker geöffnet ist. Die Kraftlinie kann beispielsweise immer parallel zur Referenzanordnungs-Ausrichtung liegen.

Gemäß einer erfindungsgemäßen Weiterbildung definiert die Referenzanordnung eine Referenzanordnungs-Ebene, die senkrecht zur funktionalen Richtung bzw. Ebene steht. Dabei kann die Kopplungsmechanik die Referenzanordnungsebene in jeder Öffnungsstellung des Instrumentenangriffs senkrecht zur funktionalen Richtung bzw. Ebene stellen, und vorteilhafter Weise definiert die Referenzanordnung die Lage der funktionalen Richtung bzw. Ebene. Mit anderen Worten kann das Navigationssystem wissen, dass bei einer bestimmten Anordnung von Markern, die auf der Referenzanordnung in einer Ebene liegen, an einer bestimmten Stelle dieser Ebene der Schnittpunkt mit der senkrechten funktionalen Richtung bzw. Ebene liegt.

Gemäß einer vorteilhaften Ausführungsvariante des erfindungsgemäßen Adapters ist die Kopplungsmechanik durch einen Federdruck vorgespannt, der die Angriffs-Kraftwirkung am Instrumentenangriff erzeugt. Es besteht dabei die Möglichkeit, dass die Kopplungsmechanik zwei einander kreuzende und im Kreuzungspunkt gelenkig verbundene Arme aufweist, die auf einer Seite des Kreuzungspunktes den Instrumentenangriff und auf der anderen Seite die Referenzanordnungs-Halterung aufweisen.

Der Instrumentenangriff kann zwei an gegenüberliegenden Seiten des Instruments anlegbare Anlagestücke, insbesondere Anlagebacken umfassen, die gelenkig an der Kopplungsmechanik gelagert sind, insbesondere an zwei gegenüberliegenden Enden der Arme. Was die Referenzanordnung-Halterung betrifft, so kann diese ein Halteelement aufweisen, das direkt mit der Referenzanordnung verbunden und federnd und gelenkig schwenkbar in der Kopplungsmechanik eingespannt ist, insbesondere zwischen gegenüberliegenden Enden der Arme. Bei einer solchen Ausgestaltung ist beispielsweise das Halteelement zwischen zwei gelenkig an den Armenden angeordneten Zugfedern eingespannt, die am anderen Ende der Arme die Angriffs-Kraftwirkung am Instrumentengriff erzeugen. Eine weitere Ausgestaltungsmöglichkeit besteht darin, das Halteelement an der von der Referenzanordnung abgewandten Seite gelenkig am Kreuzungspunkt der Arme anzubringen.

Im Weiteren wird die Erfindung anhand einer Ausführungsform näher erläutert. Sie kann alle hierin beschriebenen Merkmale einzeln sowie in jedweder sinnvollen Kombination umfassen. Ferner kann die Erfindung auch verfahrensmäßig oder verwendungsmäßig definiert werden, wobei die oben beschriebene Ausgestaltung des Adapters und die dadurch ermöglichte Anbringung einer Referenzanordnung an einem Instrument eine solche Definition bestimmen. In den beiliegenden Zeichnungen zum Ausführungsbeispiel zeigen:
- Figur 1: einen erfindungsgemäßen Adapter mit an ihm angebrachter Referenzanordnung;
- Figur 2: den Adapter sowie die Referenzanordnung aus Figur 1, angebracht an einer Knochen-Reibahle;
- Figur 3: eine detailliertere Ansicht für den Adapter, die Referenzanordnung und die Reibahle von der Stirnseite her;
- Figur 4: eine stirnseitige Ansicht, bei der der Adapter an einem Instrument mit schrägen Außenflächen angebracht ist; und
- Figur 5: eine Knochen-Reibahle mit einem erfindungsgemäßen Adapter im Arbeitsumfeld sowie im Navigationsumfeld.

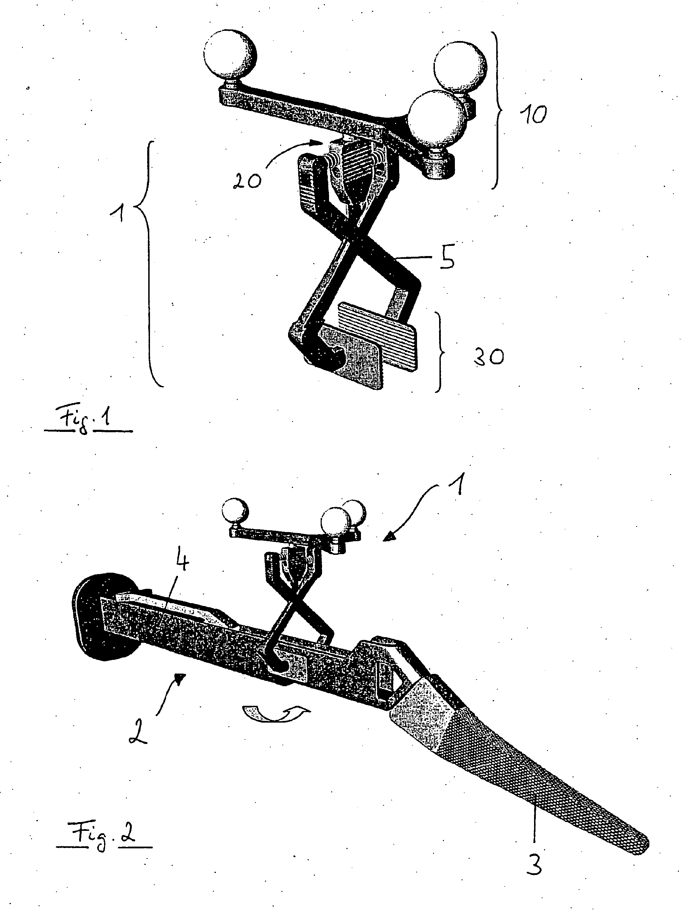

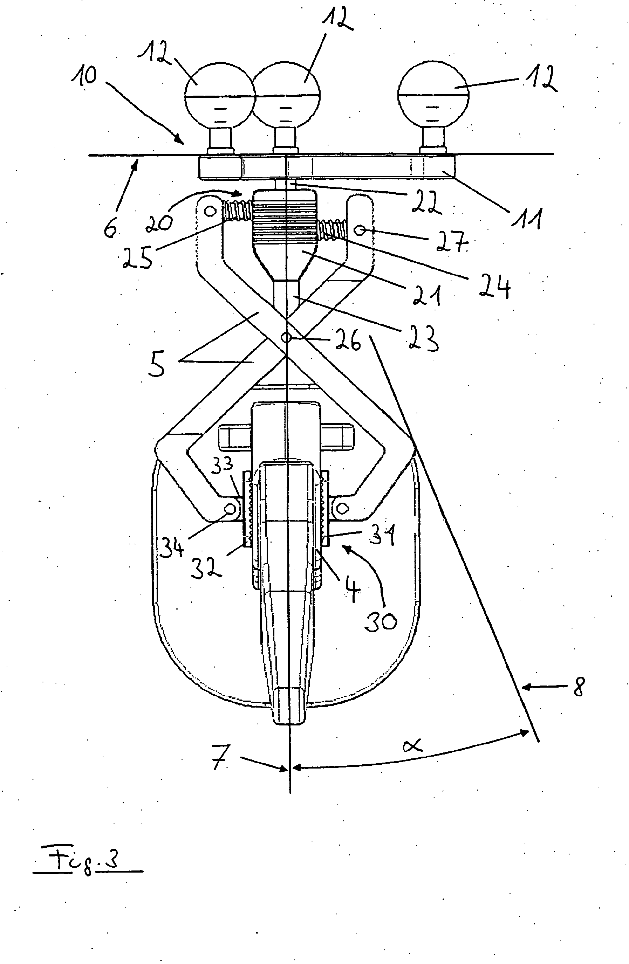

Die Figuren 1 und 2 zeigen in perspektivischer Ansicht die Hauptbestandteile eines Adapter-Anbringungssystems für Referenzanordnungen gemäß einer Ausführungsform der Erfindung. Der Klemmenadapter ist insgesamt mit dem Bezugszeichen 1 versehen, und er trägt eine Referenzanordnung 10 mit drei Reflektionsmarkern auf einem sternartigen Markerträger. In Figur 3 sind die Marker mit den Bezugszeichen 12 und der Markerträger mit dem Bezugszeichen 11 gekennzeichnet.

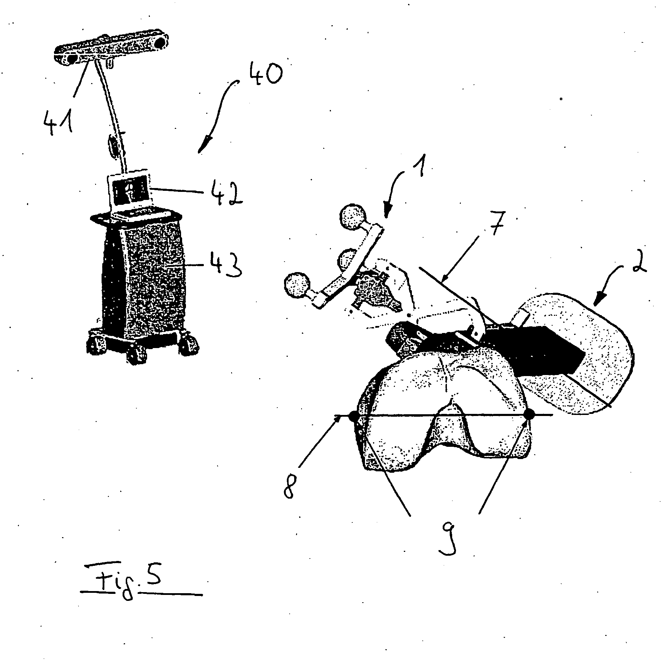

Im Großen und Ganzen weist der Adapter 1 die Referenzanordnungs-Halterung 10, den Instrumentenangriff 30 und dazwischen eine Kopplungsmechanik auf, deren Arme mit dem Bezugszeichen 5 bezeichnet sind. Der Adapter 1 wird mit der Referenzanordnung an einem Instrument angeklemmt, und er ist in diesem Zustand in Figur 2 an einer Knochen-Reibahle 2 zu sehen, deren Griff das Bezugszeichen 4 und deren Reibabschnitt das Bezugszeichen 3 tragen. In dem Zustand der Figur 2 kann die Reibahle 2 navigiert werden, beispielsweise in einem Navigationsumfeld, das in der Figur 5 zu sehen ist, mit einem Navigationssystem 40, dem ein kamerabasiertes Trackingsystem 41 zugeordnet ist und welches eine im Gehäuse 43 untergebrachte Datenverarbeitungseinheit 43 und eine Bildschirmausgabe 42 umfasst. Es wird später bei der detaillierteren Erörterung der Ebenennavigation nochmals auf die Figur 5 Bezug genommen.

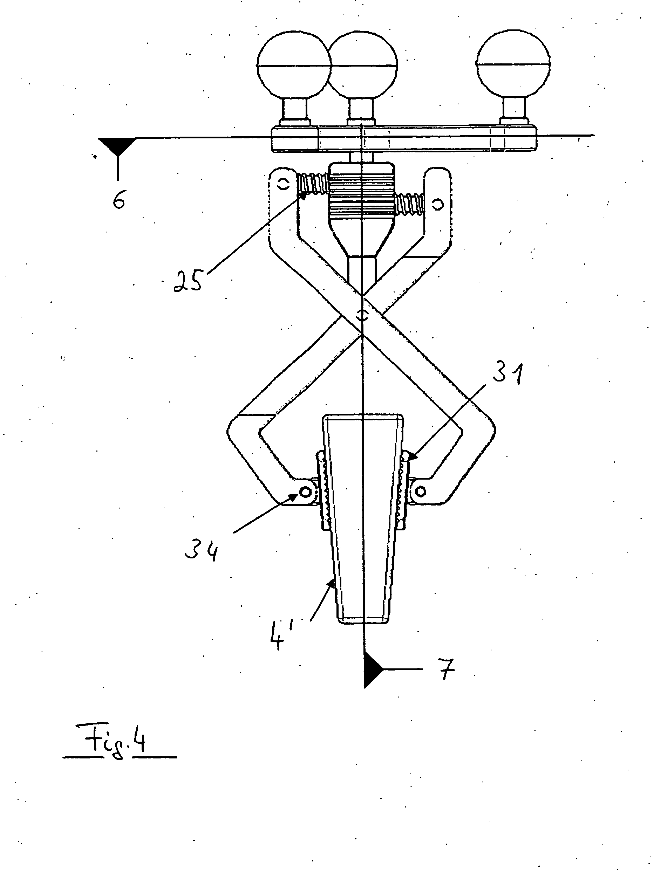

In der Figur 3 sind detailliert die Einzelheiten eines erfindungsgemäßen Anbringungssystems zu sehen, welches das Instrument (Reibahle), den Adapter und die Referenzanordnung umfasst. In der stirnseitigen Ansicht wird ersichtlich, dass der Griff 4 der Reibahle 2 gerade verlaufende Außenseiten aufweist, an denen der Instrumentenangriff 30 beidseitig angreift. Die Längsmittelebene der Reibahle ist mit dem Bezugszeichen 7 gekennzeichnet und in dieser Ansicht als Linie eingezeichnet. Der Griff 4 der Reibahle verläuft an seinen Außenseiten parallel und symmetrisch im selben Abstand zur Längsmittelebene 7. An den Seiten des Griffes 4 greifen die Angriffselemente 31, 32 an, die z.B. mit reibungsverbesserten Oberflächen versehen sind (hier geriffelte Oberflächen), um einen ausreichenden Halt zu gewährleisten. Es ist auch möglich, hier beispielsweise gummiähnliche Einsätze zu verwenden. Die Angriffselemente 31 bzw. 32 haben auskragende Fortsätze 33, die wiederum schwenkbar bei 34 an den Armen 5 des Adapters angebracht sind. Diese Schwenkbarkeit wird später noch anhand der Figur 4 diskutiert. Die Arme 5 des Adapters kreuzen sich scherenartig und sind gelenkig am Kreuzungspunkt 26 miteinander verbunden, von wo aus sie zunächst schräg und dann wieder gerade nach oben führen. Am Ende der Arme befindet sich jeweils ein weiterer Anlenkpunkt, der nur auf der rechten Seite mit 27 bezeichnet ist. Von diesen Anlenkpunkten aus erstrecken sich Zugfedern 24 und 25, die jeweils auf einer Führung gehalten werden und an dem Halteelement 21 enden.

Die Federn 24 und 25 drücken das Halteelement 21, das wiederum gelenkig am Kreuzungspunkt 26 befestigt ist, immer in eine Stellung zwischen den oberen Armteilen, bei der für jeden Öffnungswinkel der Arme 5 im Bereich des Instrumentenangriffs 30 die Ebene 6 der Referenzanordnung 10 senkrecht zur Längsmittelebene 7 verläuft. Die Referenzanordnung ist mit einem Fortsatz 22 am Halteelement 21 angebracht, und ihre Marker 12 sind auf dem Markerträger 11 aufgesetzt. Im vorliegenden Fall definiert die Oberseite des Markerträgers 11 den Verlauf der Ebene 6, d.h. der Referenzanordnungs-Ebene.

Mit anderen Worten sorgt die Kopplungsmechanik aus den Einzelteilen des Instrumentenangriffs 30, den Armen 5 und dem Halteelement 21 mit ihren gelenkigen Verbindungen und Federn 24, 25 dafür, dass die Referenzanordnungs-Ebene 6, immer senkrecht auf der Längsmittelebene 7 der Reibahle steht, und zwar egal, wieweit die Angriffselemente 31, 32 geöffnet sind.

In der Figur 3 ist noch der Anteversionswinkel α gezeigt, welcher der Winkel zwischen der Längsmittelebene 7 und der Epicondular-Achse des Oberschenkelknochens ist. Hier kann wieder auf die Figur 5 Bezug genommen werden, die aufzeigt, wie die Epicondular-Achse 8 ermittelt wird. In der Figur 5 ist das Knochenende des Femurknochens 10 zu sehen, und die Epicondular-Achse 8 wird durch die beiden Epicondylen-Punkte 9 bestimmt. Beim Ausräumen des Knochens mit der Reibahle sollte die Längsmittelebene 7 in einem vorbestimmten Verhältnis zur Epicondular-Achse 8 stehen, und mittels des Adapters 1 gemäß der vorliegenden Erfindung kann gerade diese Ebene 7 zu jedem Zeitpunkt navigiert werden, ohne dass genau die Abmessungen der Reibahle 2 bekannt sein müssen.

In der Figur 4 ist dargestellt, wie sich der erfindungsgemäße Adapter auch an Instrumente anpassen kann, die keine geradlinig bzw. parallel zur Längsmittelachse verlaufenden Seitenflächen haben. In der Figur ist wieder in einer Stirnansicht ein Instrument dargestellt, das eine sich nach untern verschmälernde Außenkontur aufweist, die aber noch symmetrisch zur Längsmittelebene 7 ist. Aufgrund des vorgesehenen Gelenkpunktes 34 für die Angriffselemente, von denen nur das linke mit dem Bezugszeichen 31 gekennzeichnet ist, passt sich der Adapter an den Verlauf der Oberflächen 4 des Instrumentengriffes an. Es ist also ein Mechanismus integriert, der den Effekt hat, dass sich beide Angriffselemente bzw. Klemmbacken symmetrisch bewegen. Das Resultat ist, dass die Längsmittelebene 7 des Griffes immer zentriert ist, und weil die Referenzanordnungs-Ebene 6 und die Längsmittelebene 7 einen rechten Winkel zueinander haben, kennt das Navigationssystem die Längsmittelebene des Instruments, wenn es die Referenzanordnung erfasst, dessen Charakteristika in einer entsprechenden Datenbank hinterlegt sind. Aus diesem Grund kann der Anteversionswinkel immer navigiert werden.

Ein möglicher Arbeitsablauf für den Einsatz einer erfindungsgemäß navigierten Reibahle beim Ausräumen eines Oberschenkelknochens ist der folgende:
Zunächst wird der Oberschenkelknochen präpariert, und danach wird der Adapter auf den Reibahlengriff aufgesetzt. Weil der Adapter in seiner Ausgestaltung dem Navigationssystem, dem ein Trackingsystem zugeordnet ist, bekannt ist, ist eine Kalibrierung der gesamten Vorrichtung nicht notwendig. Aufgrund der erfindungsgemäßen Ausgestaltung der Kopplungsmechanik und wegen der gelenkigen Anordnung der Angriffselemente (Klemmbacken) sowie der Referenzanordnungs-Halterung richtet sich der Adapter selbst auf die Mittelebene des Reibahlengriffs aus, die dann senkrecht zur Referenzanordnungs-Ebene liegt. Weil die Geometrie und die Abmessungen der Referenzanordnung dem Navigationssystem bekannt sind, ist die Bestimmung der Lage der Längsmittelebene 7 gegenüber der Referenzanordnungs-Ebene in einfacher Weise möglich.
   Im nächsten Schritt wird die Reibahle mit dem Griff auf den geplanten Anteversionswinkel ausgerichtet, und dabei nimmt der Chirurg die gesamte Vorrichtung und bringt sie zum proximalen Ende des Oberschenkelknochens, der schon zum Ausräumen vorbereitet ist. Aufgrund der erfindungsgemäß möglichen Ebenennavigation kann der Chirurg das Instrument ohne Weiteres auf den geplanten Antiversionswinkel ausrichten. Die Ebenenausrichtung genügt in diesem Fall; es muss nicht das gesamte Instrument der Geometrie und den Abmessungen nach navigiert werden.

Im Anschluss kann der Adapter abgenommen werden, um den Ausräumvorgang durchzuführen. Wenn der Adapter abgenommen ist, stört er nicht mehr bei der Ausräumarbeit, und ein erfindungsgemäßer Klemmadapter lässt sich vorteilhafterweise sehr leicht abnehmen und wieder anbringen. Eine Wiederanbringung kann jederzeit stattfinden, um den Arbeitswinkel wieder zu verifizieren, und gerade hier zeigt sich ein weiterer Vorteil der erfindungsgemäßen Adaptergestaltung.

Das Abnehmen des Adapters ist für die eigentliche Räumarbeit von Vorteil, da hier sehr große Kräfte auftreten, und es bereitet auch keine Probleme, da die Anbringung durch das Klemmen ohne weiteres wieder möglich ist. Die Verifikation und das Weiterarbeiten mit abgenommenem Adapter werden nun solange wiederholt, bis die Ausräumung ihren Endzustand erreicht hat und der korrekte, geplante Anteversionswinkel erzielt worden ist.

## Patentansprüche

1. Adapter (1) zur Anbringung einer Referenzanordnung (10) an einem medizinischen Instrument (2), das eine funktionale Instrumentenrichtung bzw. -ebene (7) aufweist, wobei der Adapter eine Referenzanordnungs-Haltefung (20) und einen Instrumentenangriff (30) aufweist, der ein größen- und/oder formanpassbarer Klemmangriff ist,
**dadurch gekennzeichnet, dass**
die Referenzanordnungs-Halterung (20) und der Instrumentenangriff (30) durch eine selbstausrichtende Kopplungsmechanik (5, 21, 24, 25, 33, 34) miteinander verbunden sind, welche beim Anbringen des Adapters (1) an dem Instrument die Referenzanordnung (10) in eine vorbestimmte Lage relativ zur funktionalen Instrumentenrichtung bzw. -ebene (7) bringt.

2. Adapter nach Anspruch 1, **dadurch gekennzeichnet, dass** die funktionale Instrumentenrichtung bzw. -ebene eine für das Instrument definierbare Richtung bzw. Ebene ist, welche die Arbeitsrichtung des Instruments bestimmt.

3. Adapter nach Anspruch 1 oder 2, **dadurch gekennzeichnet, dass** das Instrument (1) eine Knochen-Reibahle und die funktionale Ebene die Längsmittelebene der Reibahle, insbesondere des Reibahlengriffes (4) ist.

4. Adapter nach einem der Ansprüche 1 bis 3, **dadurch gekennzeichnet, dass** der Instrumentenangriff (30) zwei gegeneinander wirkende Angriffselemente (31, 32) aufweist und die Wirkrichtung des Instrumentenangriffs (30) in jeder Stellung der Angriffselemente (31, 32) in einem gleichen Positionsverhältnis zur Ausrichtung der Referenzanordnung (10) steht.

5. Adapter nach einem der Ansprüche 1 bis 4, **dadurch gekennzeichnet, dass** die Referenzanordnung (10) eine Referenzanordnungs-Ebene (6) definiert, die senkrecht zur funktionalen Richtung bzw. Ebene (7) steht.

6. Adapter nach Anspruch 5, **dadurch gekennzeichnet, dass** die Kopplungsmechanik (5, 21, 24, 25, 33, 34) die Referenzanordnungs-Ebene (6) in jeder Öffnungsstellung des Instrumentenangriffes (30) senkrecht zur funktionalen Richtung bzw. Ebene (7) stellt.

7. Adapter nach Anspruch 6, **dadurch gekennzeichnet, dass** die Referenzanordnung die Lage der funktionalen Richtung bzw. Ebene (7) definiert.

8. Adapter nach einem der Ansprüche 1 bis 7, **dadurch gekennzeichnet, dass** die Kopplungsmechanik (5, 21, 24, 25, 33, 34) durch eine Federkraft vorgespannt ist, der die Angriffs-Kraftwirkung am Instrumentenangriff (30) erzeugt.

9. Adapter nach Anspruch 8, **dadurch gekennzeichnet, dass** die Kopplungsmechanik (5, 21, 24, 25, 33, 34) zwei einander kreuzende und im Kreuzungspunkt (26) gelenkig verbundene Arme (5) aufweist, die auf einer Seite des Kreuzungspunktes (26) den Instrumentenangriff (30) und auf der anderen Seite die Referenzanordnungs-Halterung (20) aufweisen.

10. Adapter nach einem der Ansprüche 1 bis 9, **dadurch gekennzeichnet, dass** der Instrumentenangriff zwei an gegenüberliegenden Seiten des Instruments anlegbare Anlagestücke, insbesondere Anlagebacken (31, 32) umfasst, die gelenkig an der Kopplungsmechanik (5, 21, 24, 25, 33, 34) gelagert sind, insbesondere an zwei gegenüberliegenden Enden der Arme (5).

11. Adapter nach einem der Ansprüche 1 bis 10, **dadurch gekennzeichnet, dass** die Referenzanordnungs-Halterüng (20) ein Halteelement (21) aufweist, das direkt mit der Referenzanordnung (10) verbunden und federnd und gelenkig schwenkbar in der Kopplungsmechanik (5, 21, 24, 25, 33, 34) eingespannt ist, insbesondere zwischen gegenüberliegenden Enden der Arme (5).

12. Adapter nach Anspruch 11, **dadurch gekennzeichnet, dass** das Halteelement (21) zwischen zwei gelenkig an den Armenden angeordneten Zugfedern (24, 25) eingespannt ist, die am anderen Ende der Arme (5) die Angriffs-Kraftwirkung am Instrumentenangriff (30) erzeugen.

13. Adapter nach Anspruch 11 oder 12, **dadurch gekennzeichnet, dass** das Halteelement (21) an der von der Referenzanordnung (10) abgewandten Seite gelenkig am Kreuzungspunkt (26) der Arme (5) angebracht ist.

## Claims

1. An adaptor (1) for attaching a reference array (10) to a medical instrument (2) having one functional direction or plane (7) of the instrument, wherein the adaptor comprises a reference array mounting (20) and an instrument engagement (30) which is a clamping engagement which can be adjusted in size and/or in shape, **characterised in that** the reference array mounting (20) and the instrument engagement (30) are connected to each other by a self-aligning coupling mechanism (5, 21, 24, 25, 33, 34) which, when the adaptor (1) is attached to the instrument, moves the reference array (10) into a predetermined location relative to the functional direction or plane (7) of the instrument.

2. The adaptor according to claim 1, **characterised in that** the functional direction or plane of the instrument is a direction or plane which can be defined for the instrument and which determines the working direction of the instrument.

3. The adaptor according to claim 1 or 2, **characterised in that** the instrument (2) is a bone broach, and the functional plane is the longitudinal mid-plane of the broach, in particular the broach handle (4).

4. The adaptor according to any one of claims 1 to 3, **characterised in that** the instrument engagement (30) comprises two engaging elements (31, 32) which act counter to each other, and in any position of the engaging elements (31, 32), the direction of action of the instrument engagement (30) has the same positional relationship to the alignment of the reference array (10).

5. The adaptor according to any one of claims 1 to 4, **characterised in that** the reference array (10) defines a reference array plane (6) which is perpendicular to the functional direction or plane (7).

6. The adaptor according to claim 5, **characterised in that** the coupling mechanism (5, 21, 24, 25, 33, 34) places the reference array plane (6) in any opening position of the instrument engagement (30) perpendicular to the functional direction or plane (7).

7. The adaptor according to claim 6, **characterised in that** the reference array defines the location of the functional direction or plane (7).

8. The adaptor according to any one of claims 1 to 7, **characterised in that** the coupling mechanism (5, 21, 24, 25, 33, 34) is biased by a spring force which generates the engaging force action on the instrument engagement (30).

9. The adaptor according to claim 8, **characterised in that** the coupling mechanism (5, 21, 24, 25, 33, 34) comprises two mutually intersecting arms (5) which are connected in a joint at the point of intersection (26) and comprise the instrument engagement (30) on one side of the point of intersection (26) and the reference array mounting (20) on the other side.

10. The adaptor according to any one of claims 1 to 9, **characterised in that** the instrument engagement comprises two contact pieces, in particular contact jaws (31, 32), which can be applied to opposite sides of the instrument and are mounted on the coupling mechanism (5, 21, 24, 25, 33, 34) in a joint, in particular at two opposite ends of the arms (5).

11. The adaptor according to any one of claims 1 to 10, **characterised in that** the reference array mounting (20) comprises a holding element (21) which is directly connected to the reference array (10) and spring-elastically clamped such that it can pivot in a joint in the coupling mechanism (5, 21, 24, 25, 33, 34), in particular between opposite ends of the arms (5).

12. The adaptor according to claim 11, **characterised in that** the holding element (21) is clamped between two tension springs (24, 25) arranged in a joint on the arm ends and generating the engaging force action on the instrument engagement (30) at the other end of the arms (5).

13. The adaptor according to claim 11 or 12, **characterised in that** the holding element (21) is attached in a joint at the point of intersection (26) of the arms (5) on the side facing away from the reference array (10).

## Revendications

1. Adaptateur (1) pour appliquer un agencement de référence (10) sur un instrument médical (2) qui présente une direction ou un plan d'instrument fonctionnel (7), l'adaptateur comportant un support d'agencement de référence (20) et une prise d'instrument (30) qui est une prise de serrage dont la taille et/ou la forme sont adaptables, **caractérisé en ce que** le support d'agencement de référence (20) et la prise d'instrument (30) sont reliés l'un à l'autre par un mécanisme d'accouplement (5, 21, 24, 25, 33, 34) auto-orientable qui, lors de la mise en place de l'adaptateur (1) sur l'instrument, amène l'agencement de référence (10) dans une position prédéterminée par rapport à la direction ou plan d'instrument fonctionnel (7).

2. Adaptateur selon la revendication 1, **caractérisé en ce que** la direction ou plan d'instrument fonctionnel est une direction ou plan définissable pour l'instrument qui détermine la direction de travail de l'instrument.

3. Adaptateur selon l'une quelconque des revendications 1 ou 2, **caractérisé en ce que** l'instrument (1) est un alésoir pour os et le plan fonctionnel le plan médian longitudinal de l'alésoir, en particulier de la poignée d'alésoir (4).

4. Adaptateur selon l'une quelconque des revendications 1 à 3, **caractérisé en ce que** la prise d'instrument (30) comporte deux éléments de prise (31, 32) agissant l'un par rapport à l'autre, et la direction d'action de la prise d'instrument (30), dans toute position des éléments de prise (31, 32), se trouve dans un même rapport de position par rapport à l'orientation de l'agencement de référence (10).

5. Adaptateur selon l'une quelconque des revendications 1 à 4, **caractérisé en ce que** l'agencement de référence (10) définit un plan d'agencement de référence (6) qui est perpendiculaire à la direction ou plan fonctionnel (7).

6. Adaptateur selon la revendication 5, **caractérisé en ce que** le mécanisme d'accouplement (5, 21, 24, 25, 33, 34) place le plan d'agencement de référence (6), dans toute position ouverte de la prise d'instrument (30), perpendiculairement à la direction ou plan fonctionnel (7).

7. Adaptateur selon la revendication 6, **caractérisé en ce que** l'agencement de référence définit la position de la direction ou plan fonctionnel (7).

8. Adaptateur selon l'une quelconque des revendications 1 à 7, **caractérisé en ce que** le mécanisme d'accouplement (5, 21, 24, 25, 33, 34) est précontraint par une force de ressort qui produit l'action de la force de prise sur la prise d'instrument (30).

9. Adaptateur selon la revendication 8, **caractérisé en ce que** le mécanisme d'accouplement (5, 21, 24, 25, 33, 34) comporte deux bras (5) se croisant l'un l'autre et reliés de manière articulée au point de croisement (26), qui comportent sur un côté du point de croisement (26) la prise d'instrument (30) et sur l'autre côté, le support d'agencement de référence (20).

10. Adaptateur selon l'une quelconque des revendications 1 à 9, **caractérisé en ce que** la prise d'instrument comprend deux pièces de contact pouvant s'appliquer sur des côtés opposés de l'instrument, en particulier des mâchoires de contact (31, 32), qui sont supportées de manière articulée sur le mécanisme d'accouplement (5, 21, 24, 25, 33, 34), en particulier à deux extrémités opposées des bras (5).

11. Adaptateur selon l'une quelconque des revendications 1 à 10, **caractérisé en ce que** le support d'agencement de référence (20) comporte un élément de retenue (21) qui est directement relié à l'agencement de référence (10) et serré de manière à pouvoir pivoter de façon élastique et articulée dans le mécanisme d'accouplement (5, 21, 24, 25, 33, 34), en particulier entre des extrémités opposées des bras (5).

12. Adaptateur selon la revendication 11, **caractérisé en ce que** l'élément de retenue (21) est serré entre deux ressorts de traction (24, 25) disposés aux extrémités des bras, lesquels produisent, à l'autre extrémité des bras (5), l'action de force de prise sur la prise d'instrument (30).

13. Adaptateur selon l'une quelconque des revendications 11 ou 12, **caractérisé en ce que** l'élément de retenue (21) est placé, sur le côté tourné à l'opposé de l'agencement de référence (10), de manière articulée, au point de croisement (26) des bras (5).
